(19) Europäisches Patentamt
European Patent Office
Office européen des brevets

(11) Veröffentlichungsnummer: **0 300 390 B1**

(12) # EUROPÄISCHE PATENTSCHRIFT

(45) Veröffentlichungstag der Patentschrift: **29.08.90**

(51) Int. Cl.⁵: **C07C 217/78**

(21) Anmeldenummer: **88111470.6**

(22) Anmeldetag: **16.07.88**

(54) **Trifluormethyl-alkoxy-aniline, Verfahren zu ihrer Herstellung und ihre Verwendung in der Eisfarbentechnik.**

(30) Priorität: **23.07.87 DE 3724367**

(43) Veröffentlichungstag der Anmeldung:
**25.01.89 Patentblatt 89/4**

(45) Bekanntmachung des Hinweises auf die Patenterteilung:
**29.08.90 Patentblatt 90/35**

(84) Benannte Vertragsstaaten:
**CH DE FR GB IT LI NL**

(56) Entgegenhaltungen:
**DE-A- 2 254 624**
**FR-A- 745 293**
**US-A- 1 999 610**
**US-A- 2 056 899**

**CHEMICAL ABSTRACTS SERVICE REGISTRY HANDBOOK NUMBER SECTION1965-1971, Register-Nr. 35-66-5-4599-99-9**
**BEILSTEINS HANDBUCH DER ORGANISCHEN CHEMIE Auflage 4, Ergänzungswerk 3, Band 5, Teil 2, 1964, Springer Verlag, Berlin**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80(DE)**

(72) Erfinder: **Hertel, Hasso, Dr., Brunnenweg 10, D-6052 Mühlheim/Main(DE)**

EP 0 300 390 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung liegt auf dem technischen Gebiet der Zwischenprodukte und der Eisfarbenfärberei.

Die Herstellung von echten Färbungen nach den Methoden der Eisfarbentechnik durch Synthese von wasserunlöslichen Azofarbstoffen auf der Faser mittels der Reaktion einer Diazokomponente mit einer Kupplungskomponente ist in zahlreichen Veröffentlichungen seit langem beschrieben. Praktische Bedeutung hat vor allem die Bildung von Farbstoffen mit einer Kupplungskomponente aus der 2-Hydroxy-naphthalin-3-carbonsäurearylamid-Reihe erlangt (s. beispielsweise H. Rath, Lehrbuch der Textilchemie, 3. Auflage, S. 548 ff., Springer-Verlag [1972], und Melliand Textilberichte 30, 525-528 [1949]).

Von den hierfür verwendbaren Diazokomponenten sind auch einige Anilinverbindungen mit einer Trifluormethylgruppe beschrieben (s. K. Venkataraman, The Chemistry of Synthetic Dyes, Vol. I, S. 650-673, Academic Press New York (1952), und Ullmann's Encylopedia of Industrial Chemistry, 5th Ed., Vol. A3, Seiten 304–314, VHC-Verlagsgesellschaft, Weinheim [1985]), wo auch die verschiedenen Färbeweisen nach der Eisfarbentechnik unter Verwendung solcher Komponenten beschrieben sind. Solche Trifluormethylaniline sind beispielsweise 2-Chlor-5-trifluormethyl-anilin (C.I.-Nr. 37050), 4-Chlor-2-trifluormethyl-anilin (C.I.-Nr. 37055) und 2-Ethylsulfonyl-5-trifluormethyl-anilin (C.I.-Nr. 37065). Weiterhin ist aus Chem. Abstr. 52, 2412c (1958) und aus der US-Patentschrift 1 999 610 die Herstellung von Baumwollfärbungen durch Kupplung von diazotiertem 2-Methoxy-5-trifluormethyl-anilin mit 2-Hydroxy-3-naphtholsäurephenylamid gemäß der Eisfarbentechnik bekannt.

Diese Diazokomponenten liefern mit 2-Hydroxy-naphthalin-3-carbonsäurearylamid-Verbindungen als Kupplungskomponenten auf der Faser orangefarbene bis rote Färbungen mit ausreichend guten Echtheiten. Jedoch war es wünschenswert, unter Verwendung von Diazokomponenten ähnlicher Konstitution, aber leichterer Diazotierbarkeit gleich gute oder gar noch verbesserte Färbungen zu erlangen.

Es wurde nun gefunden, daß man auf der Faser nach den Methoden der Eisfarbentechnik Färbungen mit verbesserten Eigenschaften, insbesondere besseren Naßechtheiten, herstellen kann, wenn man als Diazokomponente eine Anilinverbindung entsprechend der allgemeinen Formel (1)

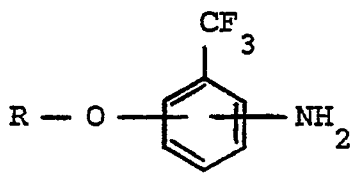

(1)

verwendet, in welcher R für eine geradkettige oder verzweigte Alkylgruppe von 2 bis 8 C-Atomen steht, die durch eine Alkoxygruppe von 1 bis 4 C-Atomen substituiert sein kann, und in welcher die Alkoxygruppe R–O– sich in para-Stellung und die Aminogruppe sich in meta-Stellung zur Trifluormethylgruppe befindet oder in welcher die Alkoxygruppe R–O– in meta-Stellung zur Trifluormethylgruppe steht und die Aminogruppe sich in ortho-Stellung zur Trifluormethylgruppe und in para-Stellung zur Alkoxygruppe befindet. Hiervon bevorzugt sind insbesondere diejenigen Verbindungen, in welchen R einen $C_2$-, $C_3$- oder $C_4$-Alkylrest bedeutet.

Die vorliegende Erfindung betrifft somit die Verwendung einer Anilinverbindung der Formel (1) als Diazokomponente in der Eisfarbenfärberei bzw. ein Verfahren zur Herstellung von wasserunlöslichen Azofarbstoffen auf der Faser, insbesondere auf der Cellulosefaser, nach den Methoden der Eisfarbentechnik, nach welcher man das mit einer Kupplungskomponente grundierte Fasermaterial in wäßrigem Medium mit der Diazoniumverbindung eines aromatischen Amins zusammenbringt und kuppelt, das dadurch gekennzeichnet ist, daß man als Diazokomponente eine Verbindung der obengenannten und definierten allgemeinen Formel (1), in Form ihrer Diazoniumverbindung, mit einer in der Eisfarbentechnik üblichen Kupplungskomponente, insbesondere einer aus der 2-Hydroxy-naphthalin-3-carbonsäurearylid-Reihe, kuppelt, wobei die Kupplungsreaktion und Farbstoffbildung auf der Faser bei einem pH-Wert zwischen 3 und 10, vorzugsweise zwischen 4 und 6, in wäßrigem Medium durchgeführt wird.

Als Kupplungskomponente dienen bevorzugt Verbindungen entsprechend der allgemeinen Formel (2)

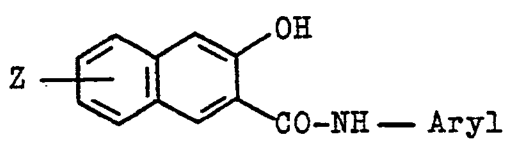

(2)

in welcher Z bevorzugt in 6- oder 7-Stellung gebunden ist und für ein Wasserstoffatom oder ein Halogenatom, wie ein Bromatom, oder eine Alkoxygruppe von 1 bis 4 C-Atomen, wie die Methoxy- oder Ethoxygruppe, steht und Aryl einen Phenylrest oder einen 1-Naphthylrest bedeutet, das durch 1, 2 oder 3 Substituenten aus der Gruppe Halogen, wie Brom und insbesondere Chlor, Nitro, Alkyl von 1 bis 4 C-Atomen, wie Ethyl und insbesondere Methyl, und Alkoxy von 1 bis 4 C-Atomen, wie Ethoxy und insbesondere Methoxy, substituiert sein kann.

In den Verbindungen der allgemeinen Formel (1) ist R beispielsweise eine Ethyl-, n-Propyl, iso-Propyl-, n-Butyl-, iso-Butyl-, sek.-Butyl-, 2-Methoxy-ethyl-, 2-Ethoxy-ethyl-, 2-Ethoxy-n-butyl-, 2-Methoxyiso-propyl-, n-Pentyl-, n-Hexyl-, n-Heptyl- oder n-Octyl-Gruppe.

Insbesondere bevorzugt ist R eine Ethyl-, n-Propyl-, iso-Propyl-, n-Butyl- oder iso-Butyl-Gruppe.

Verbindungen der allgemeinen Formel (1) sind beispielsweise: 2-Trifluormethyl-4-n-propoxy-anilin, 2-Trifluormethyl-4-(1′-methyl-ethoxy)-anilin, 2-Trifluormethyl-4-n-butoxy-anilin, 2-Trifluormethyl-4-(2′-methyl-propoxy)-anilin, 2-Trifluormethyl-4-n-pentoxy-anilin, 2-Trifluormethyl-4-(2′-methyl-butoxy)-anilin, 2-Trifluormethyl-4-(3′-methyl-butoxy)-anilin, 2-Trifluormethyl-4-n-hexoxy-anilin, 2-Trifluormethyl-4-(2′-ethoxy-butoxy)-anilin, 2-Trifluormethyl-4-n-heptoxy-anilin, 2-Trifluormethyl-4-n-octoxy-anilin, 2-Trifluormethyl-4-(2′-ethyl-hexoxy)-anilin, 5-Trifluormethyl-2-n-propoxy-anilin, 5-Trifluormethyl-2-(1′-methyl-ethoxy)-anilin, 5-Trifluormethyl-2-n-butoxy-anilin, 5-Trifluormethyl-2-(2′-methyl-propoxy)-anilin, 5-Trifluormethyl-2-n-pentoxy-anilin, 5-Trifluormethyl-2-(2′-methyl-butoxy)-anilin, 5-Trifluormethyl-2-(3′-methyl-butoxy)-anilin, 5-Trifluormethyl-2-n-hexoxy-anilin, 5-Trifluormethyl-2-(2′-ethoxy-ethoxy)-anilin, 5-Trifluormethyl-2-n-heptoxy-anilin, 5-Trifluormethyl-2-n-octoxy-anilin, 5-Trifluormethyl-2-(2′-ethyl-hexoxy)-anilin, 2-Trifluormethyl-4-ethoxy-anilin, 2-Trifluormethyl-4-(2′-methoxy-ethoxy)-anilin, 2-Trifluormethyl-4-(2′-ethoxy-ethoxy)-anilin, 2-Trifluormethyl-4-n-hexoxy-anilin, 5-Trifluormethyl-2-ethoxy-anilin, 5-Trifluormethyl-2-(1′-methyl-n-propoxy)-anilin, 5-Trifluormethyl-2-(2′-methoxy-ethoxy)-anilin, 5-Trifluormethyl-2-(2′-n-butoxy-ethoxy)-anilin und 5-Trifluormethyl-2-n-hexoxy-anilin, hiervon insbesondere bevorzugt 2-Trifluormethyl-4-ethoxy-anilin, 2-Trifluormethyl-4-n-propoxy-anilin, 2-Trifluormethyl-4-(1′-methyl-ethoxy)-anilin, 2-Trifluormethyl-4-n-butoxy-anilin, 5-Trifluormethyl-2-ethoxy-anilin, 5-Trifluormethyl-2-n-propoxy-anilin, 5-Trifluormethyl-2-(1′-methyl-ethoxy)-anilin und 5-Trifluromethyl-2-n-butoxy-anilin.

Kupplungskomponenten, wie beispielsweise solche der allgemeinen Formel (2), die in das erfindungsgemäße Verfahren eingesetzt werden können, sind beispielsweise: das Phenylamid, das 2-Methylphenylamid, das 2-Ethylphenylamid, das 2-Methoxyphenylamid, das 4-Methoxyphenylamid, das 2-Ethoxyphenylamid, das 4-Chlorphenylamid, das 4-Chlor-2-methyl-phenylamid, das 3-Nitrophenylamid, das 5-Chlor-2-methoxy-phenylamid, das 4-Methoxy-2-methyl-phenylamid, das 2,5-Dimethoxyphenylamid, das 5-Chlor-2,4-dimethoxy-phenylamid, das 4-Chlor-2,5-dimethoxy-phenylamid, das 4-Chlor-2-methoxy-5-methyl-phenylamid, das 5-Brom-2-methoxy-phenylamid und das Naphth-1-yl-amid der 2-Hydroxy-naphthalin-3-carbonsäure, das 6-Brom-2-hydroxy-naphthalin-3-carbonsäure-(2-methoxyphenyl)-amid, 6-Methoxy-2-hydroxy-naphthalin-3-carbonsäure-phenylamid und 6-Methoxy-2-hydroxy-naphthalin-3-carbonsäure-(4-chlor-2,5-dimethoxy-phenyl)-amid.

Bei den Kupplungskomponenten der allgemeinen Formel (2) bedeutet Aryl bevorzugt den Phenyl-, 2-Methyl-phenyl-, 2-Methoxy-phenyl-, 4-Methoxy-phenyl-, 2-Ethoxy-phenyl-, 4-Ethoxy-phenyl-, 4-Methoxy-2-methyl-phenyl-, 2,5-Dimethoxy-phenyl-, 4-Chlor-phenyl-, 4-Chlor-2-methyl-phenyl-, 5-Chlor-2-methoxy-phenyl-, 4-Chlor-2-methoxy-5-methyl-phenyl-, 4-Chlor-2,5-dimethoxy-phenyl-, 5-Chlor-2,4-dimethoxy-phenyl- und den 1-Naphthyl-Rest.

Die Bildung der Azofarbstoffe unter der erfindungsgemäßen Verwendung der Diazokomponente entsprechend der allgemeinen Formel (1) erfolgt gemäß den Verfahrensweisen der Eisfarbentechnik, die in der Literatur vielfach beschrieben sind (loc. cit.).

Hierfür bringt man ein Fasermaterial, insbesondere ein Cellulosefasermaterial, das zuvor mit der Kupplungskomponente alkalisch vorgrundiert wurde, mit der Diazoniumverbindung des Anilins entsprechend der allgemeinen Formel (1) in wäßrigem Medium zusammen. Dies kann allgemein so geschehen, daß man analog üblicher Methoden der Eisfarbentechnik das mit einer alkalischen wäßrigen Lösung der Kupplungskomponente grundierte Material mit einer getrennt hergestellten wäßrigen Lösung des Diazoniumsalzes, das so viel sauer-wirkende Mittel und/oder Puffersubstanzen enthält, daß sich ein pH-Bereich zwischen 3 und 10, vorzugsweise zwischen 4 und 6, einstellt und die Kupplungsreaktion sodann innerhalb dieses pH-Bereiches abläuft, zusammenbringt. Man kann aber auch die Trifluormethyl-alkoxy-anilin-Verbindungen in wäßrigem Medium in Gegenwart des grundierten Materials diazotieren, wobei gleichzeitig oder anschließend die Kupplungsreaktion durchgeführt wird; für diese Variante wird entweder die Diazotierung im schwach sauren Medium durchgeführt oder anschließend an die Diazotierung der pH auf einen für die Kupplungsreaktion günstigen Wert zwischen 3 und 10 gebracht.

Für alle diese Durchführungsformen wird zweckmäßig die Menge der bei der Diazotierung verwendeten Säure und/oder die Menge des Alkalis im grundierten Material bzw. des wäßrigen Mediums, in welchem das zu färbende, grundierte Material vorliegt, bereits zuvor so bemessen, daß, erforderlichenfalls durch Zusatz von Puffersubstanzen oder Puffergemischen, der erwünschte pH-Wert für die durchzuführende Kupplung sich gleich einzustellen vermag.

Die Diazotierung der Verbindungen (1) erfolgt bei den in der Eisfarbentechnik üblichen Temperaturen, beispielsweise bei einer Temperatur zwischen –5°C und +35°C, bevorzugt 0 bis 10°C.

Im einzelnen kann man beispielsweise wie folgt vorgehen: Die Trifluormethyl-alkoxy-anilin-Verbindung wird, vorzugsweise in verdünnter wäßriger Salzsäure, mittels einer wäßrigen Natriumnitritlösung diazotiert; danach wird überschüssige Mineralsäure mit einem säurebindenden Mittel, wie beispielsweise Natriumacetat, Natriumbicarbonat oder Natriumphosphat, gebunden und die wäßrige Diazoniumsalz-Lösung anschließend, gegebenenfalls nach Zugabe eines alkalibindenden Mittels, wie Essigsäure, Natriumdihydrogenphosphat, Borsäure oder Chromacetat, als Färbeflotte oder Färbebad verwendet. Dieses kann mittels eines Foulards auf das zu färbende Material, hier insbesondere ein Gewebe, das zuvor mit dem Alkalisalz der Kupplungskomponente imprägniert und getrocknet worden war, aufgebracht werden. Man kann diese Flotte auch in der Weise verwenden, daß man das imprägnierte, zu färbende Material, wie in Form eines Gewebes oder in Form von Fäden oder Garnen, in diese wäßrige Diazoniumsalz-Lösung als Färbebad einbringt und es darin bewegt, sei es per Hand oder sei es durch oder in entsprechende(n), in der Technik übliche(n) Apparate(n), wie Haspelkufe, Jigger oder eine(r) Jet-Apparatur. Hierbei wird der erforderliche Kupplungsbereich zwischen 3 und 10 durch entsprechende säurebindende oder alkalibindende Mittel oder Puffersubstanzen eingestellt und gehalten.

Bei der Diazotierung kann anstelle von verdünnter wäßriger Salzsäure auch verdünnte wäßrige Schwefelsäure verwendet werden, ebenfalls eine verdünnte wäßrige mittelstarke Säure, wie Phosphorsäure, Chloressigsäure, Milchsäure, Glykolsäure oder Ameisensäure. Die Diazotierung kann auch in einer wäßrigen Phase vorgenommen werden, bei der die Viskosität mittels einer üblichen Druckverdickung erhöht wurde.

Eine andere Variante der Farbstoffbildung kann nach einem Druckverfahren erfolgen, indem man die wäßrige Lösung des Diazoniumsalzes mit einem geeigneten und in der Technik üblichen Mittel, wie Stärke, Traganth, einem Celluloseether oder Celluloseester, verdickt und ein mit dem Alkalisalz der Kupplungskomponente imprägniertes Gewebe mit dieser verdickten Lösung bedruckt, wobei man auch hier einen entsprechenden Kupplungs-pH-Wert zwischen 3 und 10 durch entsprechende Wahl der säurebindenden oder alkalibindenden Mittel oder Puffersubstanzen und mit den hierfür benötigten Mengen einstellt und aufrechterhält.

Eine andere Variante des erfindungsgemäßen Verfahrens zur Herstellung von Azofarbstoffen mit einer Diazokomponente der Formel (1) und einer entsprechenden Kupplungskomponente auf der Faser besteht darin, daß man eine verdickte, zusätzlich eine schwache oder mittelstarke Säure enthaltende Emulsion oder Dispersion des Trifluormethyl-alkoxy-anilins der allgemeinen Formel (1) auf das zu färbende Gewebe, das zuvor mit dem Alkalisalz der Kupplungskomponente und Natriumnitrit imprägniert worden war, aufdruckt.

Das gefärbte Material wird anschließend, wie in der Eisfarbentechnik üblich, durch gründliches Spülen mit Wasser, alkalisches Seifen, nochmaliges Spülen mit Wasser und Trocknen fertiggestellt. Die erfindungsgemäß erhältlichen Färbungen zeigen gute Echtheitseigenschaften, insbesondere sehr gute Naßechtheiten, von denen insbesondere die Waschechtheiten bei verschiedenen Temperaturen, die Peroxidwaschechtheit, die Wasserstoffperoxidechtheit, die Sodakochechtheit, die Mercerisierechtheit, die Bleich-, Trockenreinigungs- und Reibechtheit hervorgehoben werden können.

Von den erfindungsgemäß verwendeten Trifluormethyl-alkoxy-anilin-Verbindungen der allgemeinen Formel (1) sind die Verbindungen der allgemeinen Formel (1a) und (1b)

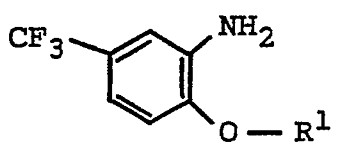

(1a)

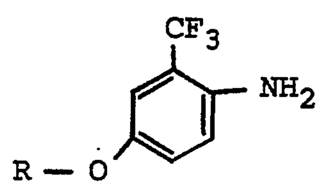

(1b)

in welcher $R^1$ eine geradkettige oder verzweigte Alkylgruppe von 3 bis 8 C-Atomen ist und R die obengenannte Bedeutung besitzt, neu.

Die Erfindung betrifft somit auch diese Verbindungen und Verfahren zu deren Herstellung. Die neuen Anilinverbindungen lassen sich analog bekannten Verfahrensweisen der Umsetzung von Chlor-nitro-trifluormethyl-benzolen mittels Alkanolen in Gegenwart von Alkalihydroxid und anschließender Reduktion der Nitrogruppe herstellen. Die vorliegende Erfindung betrifft somit auch ein Verfahren zur Herstellung der Anilinverbindungen der allgemeinen Formel (1a) und (1b), das dadurch gekennzeichnet ist, daß man eine Verbindung der allgemeinen Formel (3a) oder (3b)

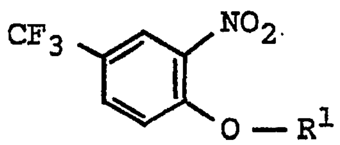

(3a)

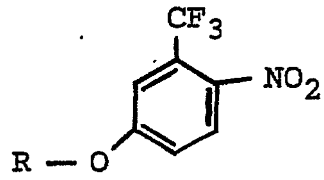

(3b)

mit einem Alkanol der allgemeinen Formel $R^1$–O bzw. R–OH mit $R^1$ und R der obengenannten Bedeutung in alkalischem Medium, vorzugsweise in wäßrig-alkalischem Medium bei einem pH-Wert von größer als 10, insbesondere bei einem pH-Wert größer als 13, und bei einer Temperatur zwischen 20 und 120°C, vorzugsweise zwischen 40 und 90°C, umsetzt und anschließend die Nitro-alkoxy-trifluormethyl-benzol-Verbindung die Nitrogruppe analog den üblichen Methoden zur Aminogruppe reduziert.

Die Umsetzung der Verbindungen der allgemeinen Formel (3) mit dem Alkanol kann mit Hilfe eines Phasentransferkatalysators, wie beispielsweise Tetramethylammoniumchlorid, Trimethyl-lauryl-ammoniumbromid, Trimethyl-cetyl-ammoniumbromid, Benzyl-dimethyl-lauryl-ammonium-chlorid oder Tetrabutyl-ammoniumhydrogensulfat, beschleunigt werden. Als alkalisch wirkendes Agenz setzt man in der Regel ein starkes Alkali, wie Natriumhydroxid oder Kaliumhydroxid, ein, in der Regel in einer Konzentration von 20 bis 70 Gew.-%, vorzugsweise von 30 bis 45 Gew.-%. Der Überschuß beträgt 50 bis 600 Gew.-%, vorzugsweise 150 bis 400 Gew.-%, bezogen auf eingesetztes Chlor-nitro-fluormethyl-benzol. Das Alkanol wird in der Regel im Überschuß eingesetzt, wie beispielsweise in einem 1- bis 9fachen, vorzugsweise 2- bis 4fachen Überschuß. Dem Reaktionsansatz kann auch ein anderes inertes organisches Lösemittel, das mit der wäßrigen Lösung teilweise oder vollständig mischbar ist, zugesetzt werden, wie beispielsweise Dimethylsulfoxid, N-Methyl-pyrrolidon, Diethylglykol oder Dimethyl-diglykol.

Zur Isolierung der Nitro-alkoxy-trifluormethyl-benzol-Verbindungen wird die organische Phase aus dem Reaktionsansatz abgetrennt; sie kann zur Entfernung von eventuell gebildeten Nebenprodukten erst mit einer verdünnten wäßrigen Alkalihydroxid-Lösung, sodann mit verdünnter wäßriger Mineralsäure, wie Salz- oder Schwefelsäure, gewaschen werden. Aus der zurückbleibenden organischen Phase destilliert man sodann überschüssiges, nicht umgesetztes Alkanol, sofern es nicht bereits in die wäßrige Phase oder die Waschlösung gegangen ist, und weiteres organisches Lösemittel ab. Das als Destillationssumpf zurückbleibende Nitro-alkoxy-trifluormethyl-benzol kann danach ohne weitere Reinigung einem Reduktionsverfahren zur Überführung der Nitrogruppe in die Aminogruppe unterworfen werden. Bevorzugt führt man die Reduktion nach Béchamp mittels Eisenpulver oder -spänen in wäßrigem Medium bei einer Temperatur zwischen 85 und 100°C durch; die Reduktion kann erfindungsgemäß auch katalytisch mit Wasserstoff mit Hilfe eines metallischen Katalysators, wie einem Palladium, Platin oder Raney-Nickel-Katalysator, unter Druck im Autoklaven erfolgen, in der Regel bei einer Temperatur zwischen 50 und 120°C und bei einem Wasserstoffdruck von 10 bis 50 bar. Die hergestellte Anilinverbindung kann aus diesem Ansatz anschließend durch fraktionierte Destillation in reiner Form erhalten werden.

Analoge Verfahrensweisen der Umsetzung von Chlornitrobenzolen mit Alkanolen in alkalischem Medium und Methoden der Reduktion von Alkoxynitrobenzolen zu deren Anilinverbindungen nach Béchamp oder auf katalytischem Wege mit Wasserstoff sind beispielsweise aus Ullmanns Encyklopädie der technischen Chemie, Band 7, Seiten 397 ff. (1974), und Band 17, Seiten 409 und 410 (1979) bekannt.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die $^1$H-NMR-Messungen erfolgten in Deuterochloroform mit Tetramethylsilan als innerem Standard.

Herstellungsbeispiel 1

a) Eine Mischung aus 225,5 Teilen 5-Chlor-2-nitro-1-trifluormethyl-benzol, 370 Teilen 1-Butanol und 500 Teilen einer wäßrigen 36%igen Kalilauge erwärmt man unter gutem Rühren auf 60°C, hält die Temperatur 4 Stunden und führt die Reaktion sodann 4 Stunden bei 70°C und 24 Stunden bei 80°C weiter. Man läßt anschließend den Ansatz auf Raumtemperatur abkühlen, trennt die hellbraune organische Phase ab, wäscht sie zweimal mit jeweils 500 Volumenteilen einer wäßrigen 3%igen Natronlauge und einmal mit 500 Volumenteilen einer wäßrigen 3%igen Salzsäure und trennt das überschüssige Butanol von der organischen Phase durch Destillation ab. Der Destillationssumpf erstarrt beim Abkühlen zu hellbraunen Kristallen.

Aus Methanol umkristallisiert erhält man blaßgelbe Kristalle des 2-Nitro-5-n-butoxy-1-trifluormethyl-benzols mit einem Schmelzpunkt von 36°C.

C,H,N-Analyse:
ber.: C 50,2%, H 4,6%, N 5,3%,
gef.: C 50,2%, H 4,0%, N 5,4%.

b) Eine Suspension von 300 Teilen Gußeisenspäne in 150 Teilen einer etwa 1,5%igen wäßrigen Salzsäure oder in einer etwa 3%igen wäßrigen Ameisensäure wird etwa 10 Minuten zum Sieden erhitzt. Zu ihr gibt man unter gutem Rühren weiterhin bei Siedetemperatur die Lösung des unter a) erhaltenen rohen 2-Nitro-5-butoxy-1-trifluormethyl-benzols in 300 Teilen Xylol. Man führt die Reduktion bei dieser Temperatur während 5 Stunden zu Ende, kühlt den Ansatz auf etwa 40°C ab, filtriert vom Eisenschlamm ab und wäscht diesen mit etwas Xylol nach. Aus der nahezu farblosen organischen Phase wird das Xylol durch Destillation entfernt und der Rückstand unter reduziertem Druck fraktioniert destilliert.

Man isoliert das 2-Trifluormethyl-4-n-butoxy-anilin bei 90°C/0,15 mbar. Es erstarrt zu farblosen Kristallen und besitzt, nach Umkristallisation aus Methanol, einen Schmelzpunkt von 46°C.

C,H,N-Analyse:
ber.: C 56,7%, H 6,0%, N 6,0%,
gef.: C 56,6%, H 6,0%, N 6,1%.
$^1$H-NMR-Analyse (δ in ppm):
0,96 (Triplett, $CH_3$); 1,47 (Sextett, $CH_2$); 1,73 (Quintuplett, $CH_2$); 3,62 (flaches Singulett, $NH_2$); 3,90 (Triplett, $CH_2$); 6,68 (Duplett, arom. H); 6.90 (Duplett, arom. H); 6,97 (Singulett, arom. H).
IR-Spektrum (in KBr; Banden bei $cm^{-1}$):
3460 (m); 3340 (m); 3240 (m); 2930 (m); 2870 (w); 1630 (m); 1500 (m); 1440 (m); 1400 (w); 1345 (m); 1290 (m); 1230 (s); 1160 (w); 1130 (m); 1100 (s); 1080 (s); 1030 (m); 1005 (w); 915 (m); 890 (w); 855 (m); 830 (m).

Herstellungsbeispiel 2

In Abänderung der Verfahrensweise des Beispieles 1 kann 2-Trifluormethyl-4-butoxy-anilin in derselben guten Qualität auch so hergestellt werden, wenn man das 1-Butanol nur in einer Menge von 148 Teilen einsetzt und die Umsetzung des Butanols mit dem 5-Chlor-2-nitro-1-trifluormethyl-benzol zunächst 4 Stunden bei 60°C, sodann 20 Stunden bei 70°C und 48 Stunden bei 80°C durchführt. Die Aufarbeitung der Butoxyverbindung und die Reduktion der Nitrogruppe erfolgt sodann gemäß der Verfahrensweise des Beispieles 1.

Herstellungsbeispiel 3

In Abänderung des Beispieles 1 kann man 2-Trifluormethyl-4-butoxy-anilin in derselben guten Qualität auch so hergestellt werden, wenn man anstelle der dort verwendeten 500 Teile 36%iger Kalilauge 375 Teile einer wäßrigen 50%igen Kaliumhydroxidlösung verwendet und die Reaktion zunächst 3 Stunden bei 50°C, sodann 3 Stunden bei 55°C, 18 Stunden bei 60°C und schießlich bei 70°C während 12 Stunden zu Ende führt. Die Aufarbeitung der Butoxyverbindung und die Reduktion der Nitrogruppe erfolgt sodann gemäß der Verfahrensweise des Beispieles 1.

Herstellungsbeispiel 4

a) Eine Mischung aus 225 Teilen 4-Chlor-3-nitro-1-trifluormethyl-benzol, 180 Teilen 2-Methyl-1-propanol und 500 Teilen einer wäßrigen 36%igen Kalilauge wird unter gutem Rühren auf 40°C erwärmt und 8 Stunden bei dieser Temperatur, zunächst unter Anwendung einer leichten Kühlung, gehalten. Danach setzt man die Umsetzung während 16 Stunden bei 50°C und nochmals 24 Stunden bei 60°C fort. Das synthetisierte 3-Nitro-4-(2'-methyl-propoxy)-1-trifluormethyl-benzol wird gemäß den Angaben des Beispieles 1a) durch Waschen der organischen Phase mit verdünnter wäßriger Natronlauge und verdünnter wäßriger Salzsäure und anschließende Abdestillation des Isobutanols als hellbrauner Rückstand erhalten.

b) Die rohe, gemäß Abschnitt a) erhaltene Nitro-Verbindung wird analog der Verfahrensweise des Beispieles 1b) reduziert. Nach Aufarbeitung erhält man das 5-Trifluormethyl-2-(2'-methyl-propoxy)-anilin mit einem Schmelzpunkt von −2°C (Kp.: 86°C/0,2 mbar).

C,H,N-Analyse:
ber.: C 56,7%, H 6,0%, N 6,0%,
gef.: C 56,5%, H 6,0%, N 6,0%.
$^1$H-NMR-Analyse (δ in ppm):
1,05 (Duplett, 2 $CH_3$); 2,13 (Nonett, CH); 3,79 (Duplett, $CH_2$); 3,86 (flaches Singulett, $NH_2$); 6,78 (Duplett, arom. H); 6,91 (Singulett, arom. H); 6,95 (Duplett, arom. H).
IR-Spektrum (Schicht zwischen NaCl-Platten; Banden bei $cm^{-1}$):
3480 (m); 3390 (m); 2950 (m); 2930 (m); 2870 (w); 1610 (m); 1515 (m); 1440 (m); 1380 (w); 1330 (s); 1290 (w); 1215 (s); 1140 (m); 1110 (s); 1020 (w); 970 (w); 915 (w); 860 (m); 800 (m).

Herstellungsbeispiel 5

a) Man erwärmt eine Mischung aus 225,5 Teilen 4-Chlor-3-nitro-1-trifluormethyl-benzol und 400 Teilen 2-Propanol auf 40°C und gibt unter gutem Rühren innerhalb von drei Stunden 500 Teile einer 36%igen wäßrigen Kalilauge zu. Man führt die Umsetzung unter Rühren während 6 Stunden bei 40°C,

sodann 40 Stunden bei 50°C und schließlich 8 Stunden bei 60°C fort und zu Ende. Anschließend kühlt man den Ansatz auf 20°C ab. Beim Verdünnen mit der doppelten Menge Wasser fällt das 3-Nitro-4-(1′-methyl-ethoxy)-1-trifluormethyl-benzol in blaßgelben Kristallen aus. Es besitzt nach Umkristallisation aus Methanol einen Schmelzpunkt von 50°C.

b) Die gemäß a) erhaltene rohe, nicht umkristallisierte Nitro-Verbindung kann direkt in die Reduktion nach Béchamp gemäß den Angaben des Beispieles 1b) eingesetzt werden. Nach analoger Aufarbeitung erhält man durch fraktionierte Destillation bei einem Siedepunkt von 54°C/0,04 mbar das 5-Trifluormethyl-2-(1′-methyl-ethoxy)-anilin, das einen Schmelzpunkt von unterhalb −15°C besitzt.

C,H,N-Analyse:
ber.: C 54,8%, H 5,5%, N 6,4%,
gef.: C 54,7%, H 5,5%, N 6,3%.

IR-Spektrum (Schicht zwischen NaCl-Platten; Banden bei $cm^{-1}$):
3520 (w); 3420 (w); 3020 (m); 2960 (w); 1530 (m); 1630 (m); 1460 (m); 1400 (w); 1350 (s); 1310 (m); 1240 (s); 1160 (m); 1130 (s); 1080 (w); 970 (m); 930 (m); 880 (m); 825 (m).

Herstellungsbeispiel 6

a) Eine Mischung aus 225,5 Teilen 4-Chlor-3-nitro-1-trifluormethyl-benzol, 200 Teilen 1-Butanol und 500 Teilen einer wäßrigen 36%igen Kalilauge erwärmt man unter gutem Rühren auf 40°C, hält die Temperatur während 8 Stunden und führt die Reaktion sodann 8 Stunden bei 50°C und 8 Stunden bei 60°C weiter. Man läßt danach den Ansatz auf Raumtemperatur abkühlen und arbeitet die organische Phase gemäß den Angaben des Beispieles 1a) auf.

Die Reduktion der Nitroverbindung kann wie im Beispiel 1b) beschrieben durchgeführt werden. Man isoliert das 5-Trifluormethyl-2-n-butoxy-anilin durch fraktionierte Destillation bei 75°C/0,06 mbar; es besitzt einen Schmelzpunkt von −8°C.

C,H,N-Analyse:
ber.: C 56,7%, H 6,0%, N 6,0%,
gef.: C 56,6%, H 6,0%, N 6,1%.

$^1$H-NMR-Analyse (δ in ppm):
0,84 (Triplett, $CH_3$); 1,50 (Sextett, $CH_2$); 1,81 (Quintuplett, $CH_2$); 3,88 (flaches Singulett, $NH_2$); 4,02 (Triplett, $CH_2$); 6,78 (Duplett, arom. H); 6,90 (Singulett, arom. H); 6,95 (Duplett, arom. H).

IR-Spektrum (Schicht zwischen NaCl-Platten; Banden bei $cm^{-1}$):
3470 (m); 3390 (m); 2950 (m); 2890 (w); 1610 (m); 1515 (m); 1440 (m); 1395 (w); 1350 (s); 1290 (m); 1215 (s); 1140 (s); 1105 (s); 1020 (m); 960 (w); 915 (m); 860 (m); 800 (m).

b) In Abänderung der oben unter Abschnitt a) beschriebenen Verfahrensweise kann 5-Trifluormethyl-2-n-butoxy-anilin in derselben guten Qualität auch so hergestellt werden, wenn man die Reduktion des rohen 3-Nitro-4-n-butoxy-1-trifluormethyl-benzols mit Wasserstoff und einem Nickelkatalysator bei einer Temperatur von etwa 50°C und einem Wasserstoffdruck von 60 bar vornimmt.

Das 5-Trifluormethyl-2-n-butoxy-anilin erwies sich im AMES-Test als nicht-mutagen.

Herstellungsbeispiel 7

a) Man erwärmt unter gutem Rühren eine Mischung aus 225,5 Teilen 5-Chlor-2-nitro-1-trifluormethyl-benzol, 230 Teilen 1-Heptanol und 500 Teilen einer wäßrigen 36%igen Kalilauge auf 50°C. Nach 2 Stunden erhöht man die Temperatur auf 60°C und hält sie bei dieser Temperatur während 8 Stunden; die Reaktion führt man sodann noch 8 Stunden bei 70°C, 8 Stunden bei 80°C und schließlich 16 Stunden bei 90°C weiter. Danach sind 5% der Ausgangs-Chlornitrobenzol-Verbindung nicht umgesetzt. Man arbeitet das synthetisierte 2-Nitro-5-heptoxy-1-trifluormethyl-benzol analog den Angaben des Beispieles 1a) durch Waschen der abgetrennten organischen Phase des Ansatzes mit verdünnter wäßriger Natronlauge und verdünnter wäßriger Salzsäure und durch Abdestillation des Heptanols auf. Der Rückstand stellt ein dunkles Öl dar.

b) Zur Reduktion der Nitrogruppe kann das Produkt von a) ungereinigt eingesetzt werden. Die Reduktion erfolgt analog den Angaben des Beispieles 1b) nach Béchamp und die Aufarbeitung erfolgt in analoger Weise. Man erhält das 2-Trifluormethyl-4-heptoxy-anilin in guter Reinheit durch fraktionierte Destillation bei einem Siedepunkt von 113°C/0,6 mbar. Smp.: 29°C.

C,H,N-Analyse:
ber.: C 61,1%, H 7,3%, N 5,1%,
gef.: C 60,9%, H 7,4%, N 5,0%.

IR-Spektrum (in KBr; Banden bei $cm^{-1}$):
3480 (m); 3400 (m); 2920 (m); 2850 (w); 1625 (m); 1500 (m); 1475 (w); 1435 (m); 1380 (w); 1340 (w); 1315 (m); 1285 (s); 1215 (m); 1160 (w); 1135 (w); 1100 (s); 1040 (m); 970 (w); 900 (w); 880 (w); 850 (w); 810 (m); 730 (w).

$^1$H-NMR-Analyse (δ in ppm):
0,88 (Triplett, $CH_3$); 1,33 (breites Singulett, 4 $CH_2$); 1,74 (Quintuplett, $CH_2$); 3,74 (Singulett, $NH_2$); 3,86 (Triplett, $CH_2$); 6,65 (Duplett, arom. H); 6,91 (Duplett, arom. H); 6,95 (Duplett, arom. H).

Herstellungsbeispiele 8 bis 16:

Gemäß der erfindungsgemäßen Verfahrensweise, wie beispielsweise analog den Angaben der obigen Ausführungsbeispiele, können auch folgenden Trifluormethyl-alkoxy-anilin-Verbindungen hergestellt werden:

| Bsp. | Verbindung |
|---|---|
| 8 | 2-Trifluormethyl-4-ethoxy-anilin (Kp.: 66°C/0,13 mbar; Fp.: 69°C) |
| 9 | 5-Trifluormethyl-2-ethoxy-anilin (Kp.: 120°C/ 20 mbar; Fp.: 51°C) |
| 10 | 2-Trifluormethyl-4-n-propoxy-anilin (Kp.: 75°C/0,1 mbar; Fp.: 49°C) |
| 11 | 5-Trifluormethyl-2-n-propoxy-anilin (Kp.: 68°C/0,06 mbar) |
| 12 | 5-Trifluormethyl-2-(1′-methyl-n-propoxy)-anilin (Kp.: 72°C/0,07 mbar) |
| 13 | 5-Trifluormethyl-2-n-pentoxy-anilin (Kp.: 95°C/0,15 mbar) |
| 14 | 2-Trifluormethyl-4-n-heptoxy-anilin (Kp.: 113°C/0,6 mbar; Fp.: 29°C) |
| 15 | 5-Trifluormethyl-2-n-octoxy-anilin (Kp.: 122°C/0,15 mbar; Fp.: 0°C) |
| 16 | 2-Trifluormethyl-4-n-octoxy-anilin (Kp.: 115°C/0,07 mbar; Fp.: 18°C) |

Färbebeispiel 1

Zur Herstellung einer Färbung eines Baumwollgewebes imprägniert man das Gewebe zunächst mit einer wäßrigen Grundierflotte, bestehend aus einer Lösung von 18 Teilen 2-Hydroxy-naphthalin-3-carbonsäure-(2′-methoxy-phenyl)-amid, 8,3 Teilen Natriumhydroxid und 5 Teilen eines handelsüblichen Schutzkolloides in 1000 Teilen Wasser. Die Flottenaufnahme soll 80% des Warengewichtes betragen. Das imprägnierte Gewebe wird bei etwa 120°C getrocknet und anschließend mit einer das Diazoniumsalz des 5-Trifluormethyl-2-n-propoxy-anilins enthaltenden wäßrigen Entwicklungsflotte bei einer Flottenaufnahme von 80%, bezogen auf das Warengewicht überklotzt. Die Entwicklungsflotte wird durch Diazotieren von 15 Teilen 2-Trifluormethyl-5-n-propoxy-anilin in 215 Teilen einer 3%igen wäßrigen Salzsäure mittels 13 Teilen einer 40%igen wäßrigen Natriumnitritlösung und nachfolgende Zugabe von 20 Teilen Natriumacetattrihydrat und 5 Teilen Essigsäure und Auffüllen der erhaltenen Diazoniumsalzlösung mit Wasser auf 1000 Teile hergestellt.

Nach dem Überklotzen mit der Entwicklungsflotte erfolgt ein Luftgang des überklotzten Gewebes von etwa 1 Minute und anschließend eine Heißwasserpassage. Die Nachbehandlung der erhaltenen Färbung erfolgt in üblicher Weise, beispielsweise durch Spülen des gefärbten Gewebes in kaltem und heißem Wasser durch kochende Behandlung während etwa 15 Minuten in einem ein nichtionogenes Waschmittel enthaltenden Bad, durch erneutes Spülen in heißem und kaltem Wasser und Trocknen.

Man erhält eine Rotfärbung von sehr guten Echtheitseigenschaften, von denen insbesondere die guten Naßechtheitseigenschaften hervorgehoben werden können.

Färbebeispiel 2

a) Es wird zunächst die folgende Grundierungsflotte hergestellt: 15 Teile 2-Hydroxy-naphthalin-3-carbonsäure-(5′-chlor-2′,4′-dimethoxy-phenyl)-amid werden mit 36 Teilen Ethanol angeteigt und durch Zugabe von 7 Teilen einer 32%igen wäßrigen Natronlauge sowie 15 Teilen Wasser von 60°C gelöst. 5 Teile einer 33%igen wäßrigen Formaldehydlösung werden zugegeben, und die so bereitete Stammlösung wird nach 10 Minuten in eine 35°C warme Lösung von 58 Teilen einer 32%igen wäßrigen Natronlauge, 120 Teilen Natriumchlorid und 18 Teilen eines handelsüblichen Fettsäure-Eiweißabbauprodukt-Kondensationsproduktes in 6000 Teilen weichem Wasser eingerührt. Mit dieser Grundierungsflotte wird in einem Kreuzspulapparat eine Kreuzspule mit 600 Teilen Baumwollgarn, das zur Entfernung von störenden Nichtcellulose-Substanzen alkalisch mit einem Tensid und mit Sequestriermittel abgekocht worden war,

30 Minuten lang durchflossen. Anschließend wird die Grundierungsflotte abgelassen und die grundierte Spule 10 Minuten lang mit einer Lösung von 240 Teilen Natriumchlorid und 4 Teilen einer 32%igen wäßrigen Natronlauge in 6000 Teilen Wasser gespült. Das Spülbad wird anschließend abgelassen.

b) Sodann wird dem Kreuzspulapparat eine Entwicklungsflotte zugeführt, die wie folgt bereitet wird: 19 Teile 4-n-Butoxy-2-trifluormethyl-anilin werden unter gutem Rühren in eine Mischung aus 80 Teilen Wasser und 29 Teilen einer 32%igen Salzsäure eingetragen. Etwa 30 Teile Eis werden zugesetzt; sodann wird das Amin unter gutem Rühren durch allmähliche Zugabe von 6,3 Teilen Natriumnitrit in konzentriert-wäßriger Lösung bis-diazotiert. Man rührt 30 Minuten nach und gießt diese Stammlösung sodann in eine Lösung aus 10,8 Teilen Essigsäure, 50 Teilen Natriumacetat-trihydrat und 12 Teilen eines Octadecylalkohol-polyglykolethers in 5800 Teilen Wasser ein. Diese Entwicklungsflotte wird 30 Minuten lang bei 20°C durch die grundierte Kreuzspule gepumpt und anschließend aus dem Apparat abgelassen. Die Kreuzspule wird sodann in üblicher Weise mit einer essigsauren wäßrigen Lösung gespült, sodann mit kaltem Wasser klargespült und zunächst bei 60°C, dann bei 100°C geseift, anschließend mit warmem und kaltem Wasser gespült und schließlich getrocknet.

Es wird eine rote Baumwollfärbung in guter Farbausbeute und mit guten Echtheiten, wie insbesondere Waschechtheiten, erhalten.

Färbeispiel 3

a) 3,5 Teile 2-Hydroxy-naphthalin-3-carbonsäure-(naphth-1'-yl)-amid werden mit einer etwa 35°C warmen Mischung aus 4,5 Teilen Ethanol, 5,5 Teilen Wasser, 2,4 Teilen einer 32%igen wäßrigen Natronlauge und 2 Teilen einer 33%igen wäßrigen Formaldehydlösung übergossen und durch Rühren gelöst. Nach etwa 10 Minuten wird diese Lösung in eine Lösung von 8,5 Teilen einer 32,5%igen wäßrigen Natronlauge und 2 Teilen eines Fettsäure-Eiweißabbauprodukt-Kondensationsproduktes in 1000 Teilen Wasser gegeben. In das so hergestellte Bad trägt man 50 Teile eines gebleichten, abgekochten und vorgenetzten Baumwollgarnes ein und bewegt es darin 30 Minuten. Es wird sodann herausgenommen und in einer Lösung aus 30 Teilen Natriumchlorid und 1,3 Teilen einer 32%igen wäßrigen Natronlauge in 1000 Teilen Wasser gespült.

b) Getrennt hiervon wird das Entwicklungsbad wie folgt hergestellt: 2 Teile 5-Trifluormethyl-2-ethoxy-anilin werden in ein Gemisch aus 20 Teilen Wasser und 4,4 Teilen einer wäßrigen 32%igen Salzsäure eingerührt. Nach einigem Nachrühren gibt man 5 Teile Eis hinzu und diazotiert mit 0,72 Teilen Natriumnitrit in konzentriert-wäßriger Lösung. Nach Beendigung der Diazotierung wird die Lösung mit Wasser auf 1000 Teile verdünnt, und es werden noch 8 Teile Natriumacetat-trihydrat sowie 1 Teil eines Alkylpolyglykolethers darin gelöst.

c) Zur Herstellung der Färbung auf dem Textilmaterial verfährt man beispielsweise wie folgt: Das gemäß a) grundierte, gespülte und von der überschüssigen Flotte durch Abquetschen befreite Garn wird in das unter b) beschriebene Entwicklungsbad eingebracht und darin 30 Minuten bei etwa 20°C behandelt. Anschließend wird das Material herausgenommen, wie üblich gespült, geseift, nochmals gespült und getrocknet.

Es wird eine rote Färbung in guter Farbausbeute mit guten Echtheiten, insbesondere sehr guten Waschechtheiten, erhalten.

Färbebeispiel 4

Zur Herstellung eines roten Druckmusters verfährt man beispielsweise wie folgt: Man grundiert ein Baumwollgewebe mit 2-Hydroxy-naphthalin-3-carbonsäurephenylamid gemäß den Angaben des Färbebeispieles 1, trocknet es und bedruckt es auf einer Film- oder Rouleaux-Druckmaschine mit einer Druckpaste, die wie folgt zubereitet wird:

In 600 Teile einer üblichen wäßrigen Druckverdickung werden 5,4 Teile Natriumnitrit und anschließend 25 Teile einer Lösung, bestehend zu 50% aus 2-n-Butoxy-5-trifluormethyl-anilin, zu 25% aus Ethylendiglykol und zu 25% aus einem Umsetzungsprodukt von Ricinusöl mit 36 Mol Ethylenoxid, eingerührt. Gesondert hiervon werden in 300 Teile einer üblichen wäßrigen Druckverdickung 18 Teile einer 85%igen wäßrigen Phosphorsäure eingerührt. Die beiden Verdickungen werden sodann gut vermischt und mit Wasser oder einer Druckverdickung auf 1000 Teile aufgefüllt.

Das mit dieser Druckpaste bedruckte Gewebe wird in üblicher Weise, wie beispielsweise in den vorherigen Färbebeispielen erwähnt, nachbehandelt und fertiggestellt. Es wird ein kräftiges rotes Druckmuster mit guten Echtheitseigenschaften erhalten.

Alternativ kann man die Druckpaste auch wie folgt bereiten: 12,5 Teile 2-n-Butoxy-5-trifluormethyl-anilin werden in ein Gemisch aus 100 Teilen Wasser und 21 Teilen einer 32%igen wäßrigen Salzsäure eingerührt und bei einer Temperatur von etwa 5°C mit 11 Teilen einer 40%igen wäßrigen Natriumnitritlösung diazotiert. 6 Teile Essigsäure und 9 Teile Natriumacetat-trihydrat werden noch zugegeben, und das Ganze wird mit Wasser und Verdickung auf 1000 Teile aufgefüllt.

Färbebeispiel 5

Man verfährt gemäß der Verfahrensweise des Färbebeispieles 1, ersetzt jedoch das 2-Hydroxy-naphthalin-3-carbonsäure-(2′-methoxy-phenyl)-amid durch die äquivalente Menge an 2-Hydroxy-naphthalin-3-carbonsäure-phenylamid oder 2-Hydroxy-naphthalin-3-carbonsäure-(2′-methyl-phenyl)-amid. Man erhält ebenfalls rote Cellulosefärbungen mit guten Echtheitseigenschaften.

Färbebeispiel 6

Man verfährt gemäß der Verfahrensweise des Färbebeispieles 1, setzt jedoch anstelle von 5-Trifluormethyl-2-n-propoxy-anilin die gleiche Menge an 5-Trifluormethyl-2-isopropoxy-anilin oder die äquivalente Menge an 5-Trifluormethyl-2-n-pentoxy-anilin ein. Man erhält rote Cellulosefaserfärbungen mit guten Echtheitseigenschaften.

Färbebeispiel 7

Man verfährt gemäß der Verfahrensweise des Färbebeispieles 3, setzt jedoch anstelle von 5-Trifluormethyl-2-ethoxy-anilin die gleiche Menge an 2-Trifluormethyl-4-ethoxy-anilin oder die äquivalente Menge an 2-Trifluormethyl-4-n-propoxy-anilin ein. Man erhält rote Färbungen des Cellulosefasermaterials mit guten Echtheitseigenschaften.

Färbebeispiel 8

Man verfährt gemäß der Verfahrensweise des Färbebeispieles 3, setzt jedoch anstelle von 3,5 Teilen 2-Hydroxy-naphthalin-3-carbonsäure-(naphth-1′-yl)-amid 3,0 Teile 2-Hydroxy-naphthalin-3-carbonsäure-(5′-chlor-2′,4′-dimethoxy-phenyl)-amid oder 1,8 Teile 2-Hydroxy-naphthalin-3-carbonsäure-(4′-chlor-2′-methoxy-5′-methyl-phenyl)-amid oder 1,6 Teile 2-Hydroxy-naphthalin-3-carbonsäure-(4′-chlor-2′,5′-dimethoxy-phenyl)-amid ein. Man erhält ebenfalls rote Färbungen des Cellulosefasermaterials mit guten Echtheitseigenschaften.

**Patentansprüche**

1. Verwendung einer Verbindung entsprechend der allgemeinen Formel (1)

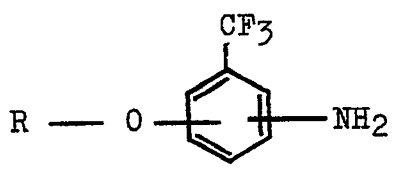

(1)

in welcher R für eine geradkettige oder verzweigte Alkylgruppe von 2 bis 8 C-Atomen steht, die durch eine Alkoxygruppe von 1 bis 4 C-Atomen substituiert sein kann, und in welcher die Alkoxygruppe R–O– sich in para-Stellung und die Aminogruppe sich in meta-Stellung zur Trifluormethylgruppe befindet oder in welcher die Alkoxygruppe R–O– in meta-Stellung zur Trifluormethylgruppe steht und die Aminogruppe sich in ortho-Stellung zur Trifluormethylgruppe und in para-Stellung zur Alkoxygruppe befindet, als Diazokomponente (Entwicklungskomponente) in der Eisfarbenfärberei.

2. Verfahren zur Herstellung von wasserunlöslichen Azofarbstoffen auf der Faser, insbesondere auf der Cellulosefaser, nach den Methoden der Eisfarbentechnik, nach welcher man das mit einer in der Eisfarbentechnik üblichen Kupplungskomponente grundierte Fasermaterial in wäßrigem Medium mit der Diazoniumverbindung eines aromatischen Amins zusammenbringt und kuppelt, dadurch gekennzeichnet, daß man als diazotierbare Diazokomponente eine Anilinverbindung der in Anspruch 1 genannten und definierten allgemeinen Formel (1) einsetzt und mit der Kupplungskomponente auf der Faser kuppelt und hierbei die Kupplungsreaktion und Farbstoffbildung auf der Faser bei einem pH-Wert zwischen 3 und 10 durchführt.

3. Eine Verbindung entsprechend der allgemeinen Formel

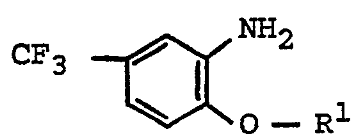

(1a)

in welcher $R^1$ für eine geradkettige oder verzweigte Alkylgruppe von 3 bis 8 C-Atomen steht.

4. Eine Verbindung entsprechend der allgemeinen Formel (1b)

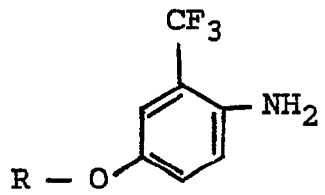

(1b)

mit R der in Anspruch 1 genannten Bedeutung.

5. Eine Verbindung nach Anspruch 4, dadurch gekennzeichnet, daß R eine $C_2$-, $C_3$- oder C4-Alkylgruppe ist.

6. Eine Verbindung nach Anspruch 4, dadurch gekennzeichnet, daß R eine Ethyl-, n-Propyl-, iso-Propyl-, n-Butyl- oder iso-Butyl-Gruppe ist.

7. Eine Verbindung nach Anspruch 3, dadurch gekennzeichnet, daß R eine $C_3$- oder $C_4$-Alkylgruppe ist.

8. Eine Verbindung gemäß Anspruch 7, dadurch gekennzeichnet, daß R eine n-Propyl-, iso-Propyl-, n-Butyl- oder iso-Butyl-Gruppe ist.

9. Verfahren zur Herstellung einer in Anspruch 3 oder 4 genannten und definierten Verbindung der allgemeinen Formel (1a) oder (1b), dadurch gekennzeichnet, daß man eine Verbindung der allgemeinen Formel (3a) oder (3b)

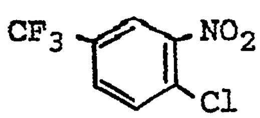

(3a)

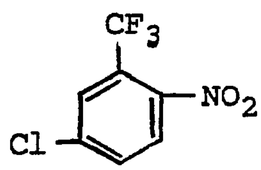

(3b)

in wäßrig-alkalischer Lösung bei einem pH-Wert von größer als 10 und einer Temperatur zwischen 20 und 120°C mit einem Alkanol der allgemeinen Formel $R^1$–OH bzw. R–OH mit $R^1$ und R der in Anspruch 3 bzw. 4 genannten Bedeutung umsetzt und anschließend die Nitrogruppe zur Aminogruppe reduziert.

**Claims**

1. The use of a compound of the general formula (1)

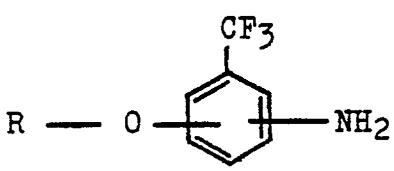

(1)

in which R is a straight-chain or branched alkyl group which has 2 to 8 carbon atoms and which can be substituted by an alkoxy group having 1 to 4 carbon atoms, and in which the alkoxy group R–O– is in the para-position and the amino group is in the meta-position relative to the trifluoromethyl group or in which the alkoxy group R-O- is in the meta-position relative to the trifluoromethyl group and the amino group is in the ortho-position relative to the trifluoromethyl group and in the para-position relative to the alkoxy group, as a diazo component (developing component) in azoic dyeing.

2. A process for producing water-insoluble azo dyes on the fiber, in particular on a cellulose fiber, by the methods of azoic dyeing technology, according to which the fiber material bottomed with a coupling component customary in azoic dyeing technology is, in an aqueous medium, brought together and coupled with the diazonium compound of an aromatic amine, which comprises using an aniline compound of the general formula (1) mentioned and defined in claim 1 as the diazotizable diazo component and coupling with the coupling component on the fiber, the coupling reaction and dye formation being carried out on the fiber at a pH between 3 and 10.

3. A compound of the general formula

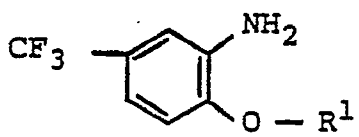

(1a)

in which $R^1$ is a straight-chain or branched alkyl group which has 3 to 8 carbon atoms.

4. A compound of the general formula (1b)

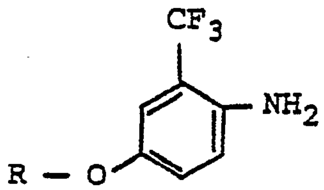

(1b)

with R being as defined in claim 1.

5. A compound as claimed in claim 4, wherein R is a $C_2$-, $C_3$- or $C_4$-alkyl group.

6. A compound as claimed in claim 4, wherein R is an ethyl, n-propyl, iso-propyl, n-butyl or iso-butyl group.

7. A compound as claimed in claim 3, wherein R is a $C_3$- or $C_4$-alkyl group.

8. A compound as claimed in claim 7, wherein R is an n-propyl, iso-propyl, n-butyl or iso-butyl group.

9. A process for preparing a compound mentioned and defined in claim 3 or 4, of the general formula (1a) or (1b), which comprises reacting a compound of the general formula (3a) or (3b)

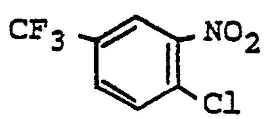

(3a)

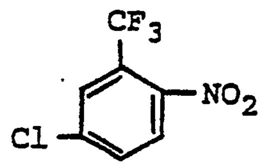

(3b)

in an aqueous-alkaline solution at a pH greater than 10 and at a temperature between 20 and 120°C with an alkanol of the general formula $R^1$–OH or R–OH, with $R^1$ and R being as defined in claim 3 or 4, and then reducing the nitro group to the amino group.

**Revendications**

1. L'emploi d'un composé de formule générale 1 ci-dessous comme composante de diazotation (composante de développement) dans la teinture des colorants à la glace:

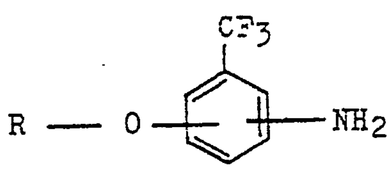

(1)

dans laquelle R désigne un alkyle à chaîne linéaire ou ramifiée en $C_2$ à $C_8$ pouvant avoir comme substituant un alcoxy en $C_1$ à $C_4$ et le groupe alcoxy R–O– est à la position para et le groupe amino à la position méta par rapport au groupe trifluorméthyle, ou bien le groupe alcoxy R–O– est à la position méta par rapport au groupe trifluorméthyle et le groupe amino à la position ortho par rapport au groupe trifluorméthyle et à la position para par rapport au groupe alcoxy.

2. Procédé de préparation sur fibres, en particulier sur des fibres cellulosiques, de colorants azoïques insolubles dans l'eau, par les méthodes de la technique des colorants à la glacé, procédé selon lequel on met en contact avec le composé de diazonium d'une amine aromatique, en milieu aqueux, la matière fibreuse qui a été piétée (apprêtée) avec une composante de copulation pour colorants à la glace, et on effectue la copulation, procédé caractérisé en ce que l'on utilise comme composante de diazotation dia-

zotable un dérivé d'aniline de la revendication 1 ci-dessus, de formule 1, que l'on copule sur la fibre avec la composante de copulation à un pH de 3 à 10 pour former le colorant.

3. Les composés de formule générale la ci-dessous,

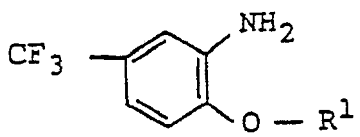

(1a)

dans laquelle $R^1$ désigne un alkyle à chaîne linéaire ou ramifiée en $C_3$ à $C_8$.

4. Les composés de formule générale (1b)

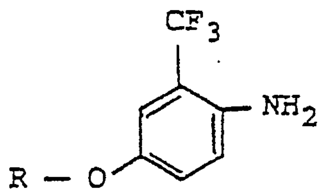

(1b)

R ayant la signification indiquée à la revendication 1.

5. Composé selon la revendication 4, caractérisé en ce que R est un alkyle à 2, 3 ou 4 atomes de carbone.

6. Composé selon la revendication 4 caractérisé en ce que R est le groupe éthyle, n-propyle, iso-propyle, n-butyle ou iso-butyle.

7. Composé selon la revendication 3 caractérisé en ce que R est un alkyle à 3 ou 4 atomes de carbone.

8. Composé selon la revendication 7, caractérisé en ce que R et le groupe n-propyle, iso-propyle, n-butyle ou iso-butyle.

9. Procédé de préparation des composés de formules (1a) et (1b) des revendications 3 et 4 précédentes, procédé caractérisé en ce que l'on fait réagir un composé de formule générale (3a) ou (3b) ci-dessous:

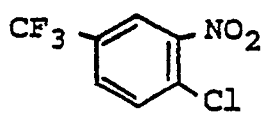

(3a)

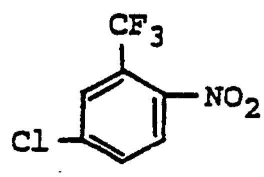

(3b)

en solution aqueuse alcaline, à un pH supérieur à 10 et à une température de 20 à 120°C, avec un alcanol $R^1OH$ ou R–OH, $R^1$ et R ayant les significations données aux revendications 3 et 4, puis on réduit le groupe nitro en groupe amino.